# EUROPÄISCHE PATENTSCHRIFT

(11) **EP 2 151 242 B1**
(45) Veröffentlichungstag und Bekanntmachung des Hinweises auf die Patenterteilung: **17.12.2014**
(21) Anmeldenummer: 09010093.4
(22) Anmeldetag: 05.08.2009
(51) Int. Cl.: A61K 31/80, A61K 33/00, A61P 1/04, A61P 31/04

(54) **Verwendung von Sauerstoff zur Behandlung von Erkrankungen der Schleimhaut des Magen-Darm-Kanals**
Use of oxygen for treatment of illnesses of the mucous membrane of the intestinal channel
Utilisation d'oxygène pour le traitement des maladies des muqueuses du canal gastro-intestinal

(30) Priorität: 06.08.2008 DE 102008037168
(43) Veröffentlichungstag der Anmeldung: 10.02.2010
(73) Patentinhaber: Nowak, Götz, 99097 Erfurt (DE)
(72) Erfinder: Nowak, Götz, 99097 Erfurt (DE)

(56) Entgegenhaltungen:
- WO-A-95/25525
- WO-A-03/066070
- WO-A-03/082392
- WO-A-03/088981
- WO-A-2005/027869
- JP-A- 62 042 922
- US-A- 4 027 045
- NOYER CHARLES M ET AL: "Hyperbaric oxygen therapy for perineal Crohn's disease" AMERICAN JOURNAL OF GASTROENTEROLOGY, Bd. 94, Nr. 2, Februar 1999 (1999-02), Seiten 318-321, XP002550573 ISSN: 0002-9270
- SEMIH GORGULU ET AL: "Hyperbaric Oxygen Enhances the Efficiency of 5-Aminosalicylic Acid in Acetic Acid-Induced Colitis in Rats" DIGESTIVE DISEASES AND SCIENCES, KLUWER ACADEMIC PUBLISHERS-PLENUM PUBLISHERS, NE, Bd. 51, Nr. 3, 1. März 2006 (2006-03-01), Seiten 480-487, XP019277152 ISSN: 1573-2568
- BUCHMAN A L ET AL: "Hyperbaric oxygen therapy for severe ulcerative colitis." JOURNAL OF CLINICAL GASTROENTEROLOGY OCT 2001, Bd. 33, Nr. 4, Oktober 2001 (2001-10), Seiten 337-339, XP008113245 ISSN: 0192-0790

## Beschreibung

Die Erfindung betrifft die Verwendung von molekularem Sauerstoff in der Behandlung der Erkrankungen der Schleimhaut des Magen-Darm-Kanals. Insbesondere betrifft die Erfindung diese Verwendung von molekularem Sauerstoff, wobei der Sauerstoff in einem flüssig-öligen linearen und/oder verzweigtkettigen Organopolysiloxan gelöst ist. Weiterhin betrifft die Erfindung pharmazeutische Präparate, umfassend Sauerstoff, ein flüssig-öliges lineares und/oder verzweigtkettiges Organopolysiloxan und gegebenenfalls weitere Hilfs- und Zusatzstoffe.

Im gesamten Verlauf des Magen-Darm-Kanals, beginnend mit der Mundhöhle bis hin zum Enddarm, werden pathologische Entzündungsvorgänge sehr häufig von anaeroben bzw. fakultativ anaeroben Keimen erzeugt. Im Bereich der Mundhöhle enthalten bereits die Recessus des entzündeten Parodonts derartige pathogene Keimspezies, im Bereich des Magens finden sich in der Tiefe der Magenschleimhautfalten magenspezifische Entzündungserreger (*Helicobacter pylori*) und ebenfalls im Zwölffingerdarm dieselben Keime, die dann lokale, aber auch unter Umständen systemische akute wie auch chronische Entzündungen hervorrufen. Der Dünndarm ist normalerweise spärlich bakterienbesiedelt, eine Reihe von fakultativen Anaerobiern kann jedoch bei einer sich rasch ausbreitenden entzündungsbedingten Keimbesiedelung des Dünndarms schwere pathologische Veränderungen der hochempfindlichen Schleimhaut des Dünndarms erzeugen. Hauptort der Bakterienflora sind der Mast- und Enddarm. Hier überwiegen zwar bereits Aerobier, aber anaerobe Vertreter sind ebenfalls in der Lage, schwerste Entzündungsvorgänge der Dickdarmschleimhaut zu induzieren bzw. langzeitig zu unterhalten. Durch den untauglichen Versuch körpereigener Reparaturmechanismen wird eine chronische Entzündungsreaktion mit leukozytärer Infiltration hervorgerufen, die nicht selten lebensbedrohliche Ausmaße beim Patienten annimmt. Die immer weiter voranschreitende Resistenzentwicklung, auch gegen die jüngsten Vertreter einer Generation von Antibiotika, führte zu neuen Wegen der Therapie. Emulsionen aus Methylpolysiloxan, u.U. in Verbindung mit Siliciumdioxid als Oberflächenträger und Emulsionsvermittler, sind in mehreren Verfahren verwendet worden. Endotoxin-bedingte Durchfallerkrankungen und Entzündungen des Zahnfleisches können mit Dimeticon behandelt werden (Patente: Futtermittelmischung. Nowak G, Fischer K. DD-WP 247 601. Diätetisches Nahrungsmittel. Nowak G, Fischer K. DD-WP 247 602. Dimeticon in der Zahnheilkunde. Nowak G, Fischer K, Merte K. P 42 07 704, 1991.)

Auch Upmeyer und Schmidt (WO 95/25525) fanden einen antibakteriellen Effekt gegen *Campylobacter pylori* und postulierten hieraus eine entsprechende Therapie.

Ferner betrifft die WO 90/07930 die Verwendung von Dimethylpolysiloxan zur Behandlung von entzündlichen Erkrankungen des Oesophagus und von entzündlichen und ulcerösen Erkrankungen des Gastrointestinaltraktes. Diese Anmeldung benutzte die gleichen Zubereitungen und Dosierungen wie die bereits zugelassenen Dimeticon-Spezialitäten. Eine Erweiterung der bisherigen Erkenntnisse, die in DD-WP 247 601 und DD-WP 247 602, aber auch in P 42 07 704 dargestellt waren, sind in dieser Schrift nicht zu finden.

WO 03/082392 offenbart die Behandlung von unter anderem Schleimhauterkrankungen wie Ulcus duodeni und Morbus Crohn mit Sauerstoff anreichernden Verbindungen. Diese Verbindungen können Sauerstoff tragende Agentien sein, wie unter anderem mit Sauerstoff angereichertes Wasser oder synthetische Verbindungen, insbesondere Perfluorkohlenwasserstoffe.

Der vorliegenden Erfindung liegt die Aufgabe zugrunde, eine einfache Substanz bei der Behandlung der Erkrankungen der Schleimhaut des Mundes und des Magen-Darm-Kanals zur Verfügung zu stellen. Das zu verwendende Präparat soll hoch wirksam, gut verträglich und für den Patienten sicher handhabbar sein. Ferner soll es keine unerwünschten Nebenwirkungen aufweisen und mit einer hohen Patientencompliance verbunden sein.

Der Schutzumfang der Erfindung ist durch die angehängten Ansprüche definiert.

Erfindungsgemäß wird diese Aufgabe durch die Verwendung von molekularem Sauerstoff gemäß der Ansprüche bei der Behandlung der Erkrankungen der Schleimhaut des Magen-Darm-Kanals, die mit einer Besiedelung mit anaeroben oder fakultativ anaeroben Krankheitserregern einhergehen, gelöst. Dabei wird die antimikrobielle Wirkung von molekularem Sauerstoff ausgenutzt, der vor allem für anaerobe und fakultativ anaerobe Mikroorganismen ein letal-toxisches Agens darstellt.

Auf eine sehr einfache Weise kann der Sauerstoff in den Magen-Darm-Kanal eingebracht werden, wenn er in einem Trägermedium gelöst ist. Hierfür eignet sich prinzipiell jedes physiologisch verträgliche Medium, das eine entsprechende Menge an Sauerstoff aufnehmen kann. Bevorzugt ist dieses Medium jedoch ein flüssig-öliges lineares und/oder verzweigtkettiges Organopolysiloxan, das entweder Methylgruppen bzw. Methyl- und Phenylgruppen und/oder Hydroxygruppen am Sliciumatom substituiert enthält. Bevorzugt verwendet werden lineare bzw. verzweigtkettige Methylpolysiloxane, bevorzugt Methylpolysiloxane und Dimethylpolysiloxane. Die Viskosität ist nicht kritisch, solange es sich um flüssige, d.h. handhabbare Öle handelt. Bevorzugt beträgt jedoch die Viskosität der verwendeten Lösung ≤ 200cSt. Es wurde überraschenderweise festgestellt, dass Organopolysiloxane in der Lage sind, erstaunlich große Mengen elementaren Sauerstoffs zu binden. So übersteigt beispielsweise die Gasbindungskapazität des Methylpolysiloxans bei weitem die physikalische Lösung dieser Gase in Flüssigkeiten und stellt somit eine ideale Transportform für Sauerstoff durch assoziatives Bindungsverhalten gegenüber den langkettigen Methylpolysiloxanen dar.

Während N₂ und Edelgase in relativ geringer Menge assoziativ gebunden werden, ist die Bindungskapazität für CO₂ größer, am stärksten wird jedoch O₂ an Methylpolysiloxane gebunden. Für Sauerstoff beträgt sie etwa 80g pro kg Methylpolysiloxan.

Durch diese physikalische Bindung wird der Sauerstoff sowohl durch die oberflächenaktive Wirkung ("Detergent") der Polysiloxane als auch durch die Eigenschaft der Methylpolysiloxanketten, an apolaren (hydrophoben) Bindungsstellen von Zellmembranen und Bakterienoberflächen am Wirkort zu adhärieren, zu den Wirkorten an der Bakterienmembran transportiert und hat wegen der "Sauerstoff-Giftigkeit" antibakterielle Effekte. Besonders trifft dies für anaerobe bzw. fakultativ anaerobe Keime zu, die bereits bei geringen Sauerstoffkonzentrationen in ihrem Mikroenvironment nicht mehr lebensfähig bzw. vermehrungsfähig sind.

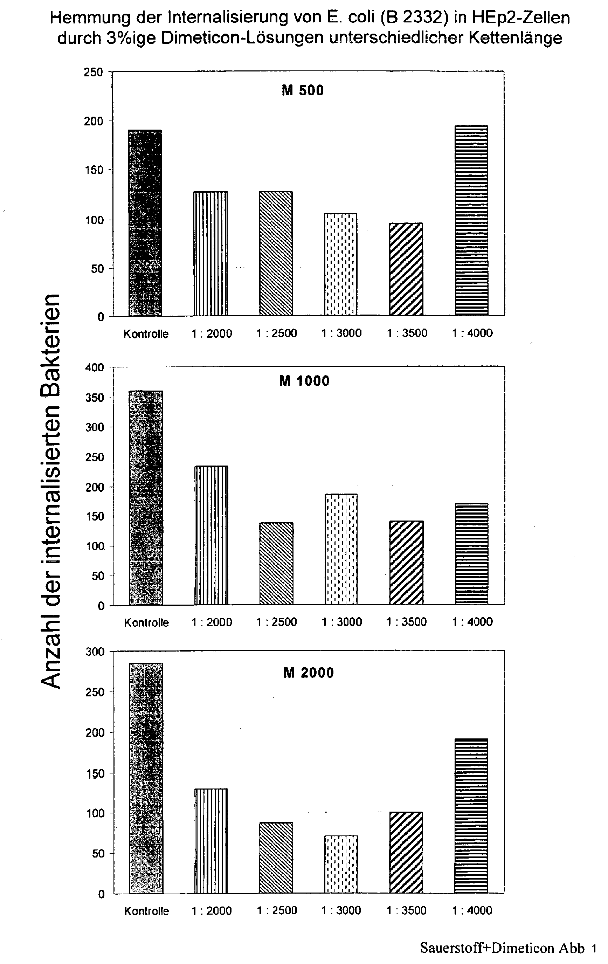

Dazu kommt die bemerkenswerte Polysiloxanwirkung der Adhärenzhemmung zwischen Wirtszellen und Keimen. In Abb. 1 ist die Adhärenzhemmung von *E. coli* an HEP-2-Zellen in Abhängigkeit von der Kettenlänge der verwendeten Polysiloxane dargestellt. Die von uns gefundenen spezifischen Wirkungen im Mikroenvironment der Keim-Wirtszellen-Interaktion sind aufgrund der O₂-Vehikelfunktion des Silikonöls zu erklären und stellen somit einen eigenen überraschenden Effekt neben der eigentlichen Wirkung des Dimeticons dar. Die molekulare Präsentation von Sauerstoffspezies, die eine sauerstoffbedingte bakterizide Wirkung haben, ist der eigenständige Wirkmechanismus, die Dimeticonoberflächenausbreitung ist der Transportmechanismus zum molekularen Wirkort der Bakterienmembran.

Ein pharmazeutisches Präparat umfasst Sauerstoff und ein oder mehrere flüssig-ölige lineare und/oder verzweigtkettige Organopolysiloxane, gegebenenfalls zusammen mit weiteren Hilfs- und Zusatzstoffen. Üblicherweise werden Organopolysiloxan-Emulsionen mit Wasser verwendet. Bevorzugt beträgt der Organopolysiloxan-Anteil etwa 5%, bevorzugter 10%, besonders bevorzugt 25%. Aber auch geringerprozentige Organopolysiloxan-Emulsionen sind wirksam.

Das Präparat kann weiterhin Hilfs- und Zusatzstoffe wie beispielsweise Farbstoffe, Konservierungsstoffe und Geschmackskorrigentien enthalten. Der Sauerstoff wird in den Präparaten entweder physikalisch gelöst, beispielsweise durch die Äquilibrierung der wässrigen Emulsion von Methylpolysiloxan mit gasförmigem Sauerstoff unmittelbar vor der Konfektionierung, oder durch Begasung der Verwendungslösung unmittelbar vor der Einnahme des Präparats. Hierzu sind entsprechende Applikatoren geeignet. Dies könnte z.B. durch eine in die Arzneimittelverpackung eingearbeitete Gaspatrone mit Dosierungsautomatik erfolgen. Bei der Verwendung von oberflächenaktiviertem Dimeticon, welches in Kapseln oder Blister eingehüllt ist, kann die Sauerstoffbindung bereits vor der Einfüllung in die entsprechende Darreichungsform mit dem Öl erfolgen, d.h. komplett mit Sauerstoff gesättigt werden. Auch die Zumischung von 0,5-2% Wasserstoffperoxidlösung oder von Sauerstoff speichernden Verbindungen, wie Häm-Proteinen oder Perfluorcarbonen, ist verwendbar.
Die bevorzugte Arzneiform ist jedoch die wässrige Emulsion der Dimeticone. Hier wird bereits während der Zubereitung der Emulsion sauerstoffgesättigtes Dimeticon verwendet. Der erste Emulgierungsschritt ist mit einer intensiven Vermischung eines bevorzugt pflanzlichen Emulgators mit der Ölphase und der nachfolgenden Einarbeitung in die wässrige Phase verbunden, in der auch Ultraschallapplikationen und Temperaturerhöhungen zur Emulgationsverbesserung benutzt werden. Es sollte sichergestellt werden, dass die Emulgierung unter Sauerstoffatmosphäre durchgeführt wird. Dadurch ist gewährleistet, dass eine maximale physikalische Bindung von Sauerstoff in die Dimeticonketten erfolgt. Der Gehalt an Sauerstoff sollte mindestens 5%, berechnet auf den Polysiloxananteil der Lösung, betragen. Die maximale Bindung ist bei etwa 7-8% Sauerstoff im Ölanteil erreicht. Die Sauerstoffbindung in der öligen Phase ist übrigens relativ stabil und wird nicht mit dem umgebenden Emulsionsträger, in der Regel Wasser, ausgetauscht. Die Sauerstoffmoleküle werden spezifisch an den Polysiloxananteil angereichert assoziativ gebunden.

Derartige sauerstoffgesättigte Lösungen sind mindestens 60 Monate haltbar und können in gasdichten Verpackungen wie beispielsweise Glasflaschen, Metallbehältnissen oder Kunststoffbehältnissen an den Endverbraucher zur direkten Verwendung abgegeben werden.

Erfindungsgemäß wird der Sauerstoff gemäß der Ansprüche bei der Behandlung der Erkrankungen der Schleimhaut des Magen-Darm-Kanals, die mit einer Besiedelung von anaeroben oder fakultativ anaeroben Krankheitserregern einhergehen, verwendet. Hierbei können alle Schleimhäute des Magen-Darm-Kanals betroffen sein, beispielsweise die Mundschleimhaut, die Rachenschleimhaut, die Schleimhaut der Speiseröhre, die Schleimhaut des Magens oder die Schleimhäute der einzelnen Darmabschnitte.

Beispiele für obligate Anaerobier sind Krankheitserreger der Gattung *Clostridium, Pseudomonas* und *Campylobacter.* Beispiele für fakultative Anaerobier sind Krankheitserreger der Gattung *Neisseria* und *Escherichia.* Beispiele für Bakterien, die erfindungsgemäß erfolgreich bekämpft werden können, sind *Helicobacterpylori* und spezielle Spezies von *E*. *coli*, aber auch *Serratia*, *Salmonella*, Yersinien, Shigellen, Staphylokokken, *B. cereus* und Vibrio. Beispiele der Erkrankungen, die erfolgreich mit molekularem Sauerstoff therapiert werden können, sind verschiedene Formen der Oesophagitis, Ulcus ventriculi und duodeni, Gastritis, Morbus Crohn, Colitis ulcerosa, aber auch unspezifische bakterielle oder virale Durchfallerkrankungen, wie die Traveller's Disease u.a.

Das pharmazeutische Präparat wird bevorzugt oral, rektal oder durch eine operativ angelegte Darmfistel lokal in den Darm verabreicht. Es kann in allen hierfür geeigneten pharmazeutischen Formen vorliegen, wie beispielsweise einer Lösung, einer Suspension, einer Emulsion, eines Gels, Hart- oder Weichgelatinekapseln oder eines Granulats. Die jeweilige Dosierung wird vom Arzt in Abhängigkeit von der Verabreichungsform, der Schwere der Erkrankung und des Zustands des Patienten festgelegt. Bevorzugt beträgt die Dosierung 3 x täglich 3-5ml einer 5-25%igen Emulsion bzw. 3-5mal täglich 1-2 Gelatinekapseln.

Wie aus den nachstehenden Beispielen hervorgeht, ist die Verwendung von molekularem Sauerstoff bei der Behandlung der vorstehend genannten Erkrankungen wirkungsvoll und zeigt keine Nebenwirkungen. Dimeticon und Sauerstoff bzw. die Kombination aus Dimeticon und Sauerstoff stellen mithin erstmals Therapeutika mit einem effektiven Wirkungsmechanismus ohne jegliche Nebenwirkungen dar. Die Erfindung wird anhand der nachstehenden Beispiele näher erläutert.

### Herstellungsverfahren für Silikonölemulsionen:

| | |
|---|---|
| Silikonöl M500 (M10, M20, M100) | 35% |
| Polaxamer 188" | 2% |
| Wasser (Phosphatpuffer 10mM) | ad 100% |

- *statt Polaxamer auch Tween 80, Cremophor EL, Span 80, HLB 8.5, Lecithin, S 570, S1570, L 595, M 1695 oder HPMC als Emulgator benutzbar.
- Das Silikonöl M500 (M10-M100) wird vor Verwendung zur Emulsionsherstellung mit O₂ (medical grade) bei 2,5 bar in einem Druckgefäß äquilibriert unter permanentem Schütteln für 2 h. Die Emulgierung erfolgt mit dem Ultraturrax 2 x 2,5 min (Stufe 4-8)
- Literatur: Yonekura Km Hayakawa K et al.: Preparation of stable silicone oil emulsion in the presence of hydroxypropyl methyl cellulose. Langmuir 1998; 14:3145-3148

### Herstellung von Dimeticon 25% oral:

4l Silfar^{®} SE 4 (35% Siliconölemulsion, Wacker Chemie AG München), zugelassen als Lebensmitteihilfsstoff und zur medizinischen Präparateherstellung, werden mit 1I Aqua bidestillata verdünnt und anschließend 2 Stunden mit medizinischem Sauerstoff (Linde) begast, danach in Polyethylenflaschen verfüllt und luftdicht verschlossen.

In ersten Anwendungen des Dimeticon 25% oral-Präparats wurden z.B. folgende Patienten behandelt:
- J.B., 73 Jahre, weibl., Diagnose: rezidivierende Gastritis, nach 2 Tripel-Therapie-Zyklen weiter Helicobacter-positiv getestet. Behandlung mit Dimeticon 25% oral 3 x 1 TL tgl., nach 6 Wochen beschwerdefrei, Helicobacter neg., keine Rezidive bis heute (Behandlung erfolgte vor 6 Jahren)
- A. St., 71 Jahre, männl., Diagnose: chronische Gastritis, Ulcus ventriculi, Zustand nach Magenresektion 1988, erfolglose dreimalige Eradiaktionstherapie. Behandlung mit Dimeticon 25% oral: nach 4 Wochen beschwerdefrei, Helicobacter neg., keine Rezidive seit 9 Jahren
- U. B., 60 Jahre, männl., Diagnose: Colitis ulcerosa, seit 11 Jahren Behandlung akuter Schübe mit Salufalk^{®} + Prednisolon, ansonsten nur Salufalk^{®}, persistierende Colitis ohne Heilungsverlauf. Behandlung mit Dimeticon 25% oral seit 4 Jahren 3 x 1 TL tgl. als Basistherapie. Nach 3 Jahren völlig beschwerdefrei. Bei Colonspiegelung Narbenbildung ohne akute Entzündungserscheinungen. Seit 2 Monaten als "geheilt" eingestuft.
- C. B., 19 Jahre, männl., Diagnose: Morbus Crohn. Nach Erstschub mit Dimeticon 25% oral 2 Jahre behandelt, nach ½ Jahr keine weiteren akuten Schübe, beschwerdefrei, nach Rektoskopie keine frischen Entzündungsherde mehr. Erhaltungsdosis 3 x 1 TL tgl.

## Patentansprüche

1. Molekularer Sauerstoff (O₂), der in einem flüssig-öligen linearen und / oder verzweigtkettigen Organopolysiloxan-Trägermedium gelöst ist, zur Verwendung in der Behandlung der Erkrankungen der Schleimhaut der Magen-Darm-Kanals, die mit einer Besiedlung von anaeroben oder fakultativ anaeroben Krankheitserregern einhergehen, **dadurch gekennzeichnet, dass** die Erkrankung eine Oesophagitis, Ulcus ventriculi oder duodeni, Gastritis, Morbus Crohn oder Colitis ulcerosa ist.

2. Molekularer Sauerstoff zur Verwendung nach Anspruch 1, **dadurch gekennzeichnet, dass** das Organopolysiloxan zusätzlich Sauerstoffträger enthält.

3. Molekularer Sauerstoff zur Verwendung nach einem der Ansprüche 1 und 2, **dadurch gekennzeichnet, dass** die anaeroben oder fakultativ anaeroben Krankheitserreger aus der Gattung Helicobacter, Vibrio, Clostridium oder Neisseria stammen.

4. Verwendung von molekularem Sauerstoff, der in einem flüssig-öligen linearen und / oder verzweigtkettigen Organopolysiloxan-Trägermedium gelöst ist, zur Herstellung eines Medikaments, das in der Behandlung der Erkrankungen der Schleimhaut des Magen-Darm-Kanals, die mit der Besiedlung von anaeroben oder fakultativ anaeroben Krankheitserregern einhergehen, **dadurch gekennzeichnet, dass** die Erkrankung eine Oesophagitis, Ulcus ventriculi oder duodeni, Gastritis, Morbus Crohn oder Colitis ulcerosa ist.

5. Verwendung nach Anspruch 4, **dadurch gekennzeichnet, dass** das Organopolysiloxan zusätzlich Sauerstoffträger enthält.

6. Verwendung nach einem der Ansprüche 4 und 5, **dadurch gekennzeichnet, dass** der anaerobe oder fakultativ anaerobe Krankheitserreger aus der Gattung Vibrio, Helicobacter, Clostridium oder Neisseria ausgewählt ist.

7. Pharmazeutisches Präparat umfassend molekularen Sauerstoff nach einem der Ansprüche 1 bis 3, zur Verwendung nach einem der Ansprüche 1 bis 3 **dadurch gekennzeichnet, dass** es in einer für die orale, enterale oder rektale Verabreichung geeigneten Form vorliegt.

## Claims

1. Molecular oxygen (O₂) which is dissolved in a liquid-oily linear and/or branched-chain organopolysiloxane carrier medium for use in the treatment of diseases of the mucous membrane of the gastro-intestinal tract which are associated with population by anaerobic or facultative-anaerobic pathogens, **characterized in that** the disease is an oesophagitis, ulcus ventriculi or duodeni, gastritis, Crohn's disease or colitis ulcerosa.

2. Molecular oxygen for use according to Claim 1, **characterized in that** the organopolysiloxane additionally contains oxygen carriers.

3. Molecular oxygen for use according to one of Claims 1 and 2, **characterized in that** the anaerobic or facultative-anaerobic pathogens are from the genus Helicobacter, Vibrio, Clostridium or Neisseria.

4. Use of molecular oxygen which is dissolved in a liquid-oily linear and/or branched-chain organopolysiloxane carrier medium for preparing a medicament which is in the treatment of the diseases of the mucous membrane of the gastro-intestinal tract which are associated with population by anaerobic or facultative-anaerobic pathogens, **characterized in that** the disease is an oesophagitis, ulcus ventriculi or duodeni, gastritis, Crohn's disease or colitis ulcerosa.

5. Use according to Claim 4, **characterized in that** the organopolysiloxane additionally contains oxygen carriers.

6. Use according to one of Claims 4 and 5, **characterized in that** the anaerobic or aerobic pathogen is selected from the genus Vibrio, Helicobacter, Clostridium or Neisseria.

7. Pharmaceutical preparation comprising molecular oxygen according to one of Claims 1 to 3 for use according to one of Claims 1 to 3, **characterized in that** it is present in a form which is suitable for oral, enteral or rectal administration.

## Revendications

1. Oxygène moléculaire (O₂) dissous dans un matériau porteur à base d'organopolysiloxane linéaire et/ou ramifié liquide huileux, destiné à une utilisation pour le traitement des maladies des muqueuses du canal gastro-intestinal accompagnées d'une colonisation de pathogènes anaérobies ou éventuellement aérobies, **caractérisé en ce que** la maladie est une oesophagite, un ulcère gastrique ou duodénal, une gastrite, la maladie de Crohn ou une colite ulcéreuse.

2. Oxygène moléculaire destiné à une utilisation selon la revendication 1, **caractérisé en ce que** l'organopolysiloxane contient en outre des oxydants.

3. Oxygène moléculaire destiné à une utilisation selon l'une quelconque des revendications 1 et 2, **caractérisé en ce que** les pathogènes anaérobies ou éventuellement aérobies sont issus du genre Helicobacter, Vibrio, Clostridium ou Neisseria.

4. Utilisation d'oxygène moléculaire dissous dans un matériau porteur à base d'organopolysiloxane linéaire et/ou ramifié liquide huileux, pour la fabrication d'un médicament pour le traitement des maladies des muqueuses du canal gastro-intestinal accompagnées d'une colonisation de pathogènes anaérobies ou éventuellement aérobies, **caractérisée en ce que** la maladie est une oesophagite, un ulcère gastrique ou duodénal, une gastrite, la maladie de Crohn ou une colite ulcéreuse.

5. Utilisation selon la revendication 4, **caractérisée en ce que** l'organopolysiloxane contient en outre des oxydants.

6. Utilisation selon l'une quelconque des revendications 4 et 5, **caractérisée en ce que** les pathogènes anaérobies ou éventuellement aérobies sont choisis dans le genre Vibrio, Helicobacter, Clostridium ou Neisseria.

7. Préparation pharmaceutique comprenant de l'oxygène moléculaire selon l'une quelconque des revendications 1 à 3 pour une utilisation selon l'une quelconque des revendications 1 à 3, **caractérisée en ce qu'**elle se présente sous une forme appropriée pour l'administration orale, entérale ou rectale.
